(19) Europäisches Patentamt
European Patent Office
Office européen des brevets

(11) Numéro de publication : **0 147 279**
**B1**

(12) **FASCICULE DE BREVET EUROPÉEN**

(45) Date de publication du fascicule du brevet : **04.03.87**

(51) Int. Cl.[4] : **C 07 C 89/02**, C 07 C 91/12

(21) Numéro de dépôt : **84402519.7**

(22) Date de dépôt : **06.12.84**

(54) **Nouveau procédé de préparation de diamine-alcools.**

(30) Priorité : **21.12.83 FR 8320486**

(43) Date de publication de la demande : **03.07.85 Bulletin 85/27**

(45) Mention de la délivrance du brevet : **04.03.87 Bulletin 87/10**

(84) Etats contractants désignés : **AT BE CH DE FR GB IT LI LU NL SE**

(56) Documents cités :
**FR-A- 2 301 235**
**GB-A- 275 622**
**GB-A- 276 012**
**US-A- 3 432 553**

(73) Titulaire : **Laboratoires Pharmascience**
**73, blvd. de la Mission Marchand**
**F-92400 Courbevoie (FR)**

(72) Inventeur : **Mercier, Martine**
**Chemin du puits mignon St Martin de Nigelles**
**F-28130 Maintenon (FR)**
Inventeur : **Rancurel, Alain**
**3, rue d'Ouarville Leves**
**F-28300 Mainvilliers (FR)**

(74) Mandataire : **Warcoin, Jacques et al**
**Cabinet Régimbeau 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 147 279 B1

**Description**

La présente invention concerne un procédé de préparation de diamine-alcools.

Plus particulièrement la présente invention concerne des procédés de préparation de diamine-alcools de formule :

$$R—NH—CH_2—CH(OH)—CH_2NH_2$$

dans laquelle R est un radical alcoyle en $C_1$ à $C_{30}$, plus particulièrement un radical alcoyle non ramifié en $C_5$ à $C_{18}$.

Les composés en cause sont englobés dans la formule générale des composés décrits dans le brevet américain 4 196 217.

Dans ce brevet, qui, outre les diamino-alcools concernent essentiellement les éthers amino alcools, on propose un procédé de préparation mettant en œuvre une méthode dite méthode de Gabriel qui passe par l'intermédiaire d'un phtalimide.

Ce procédé peut être schématisé comme suit par analogie avec le procédé décrit dans le brevet américain pour les éthers :

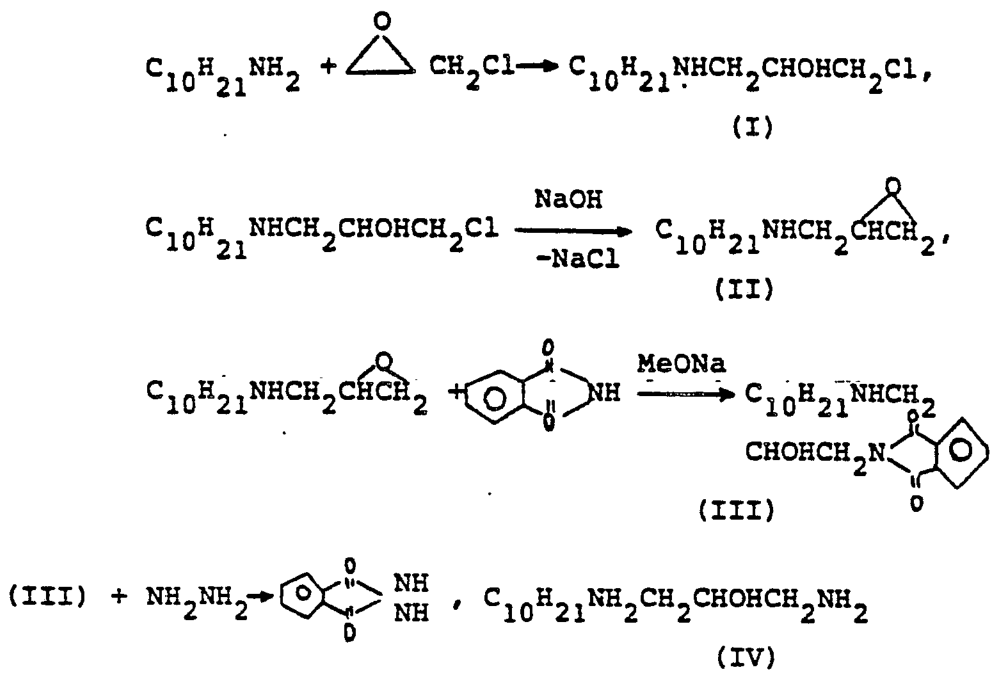


(I) (II) (III) (IV)

$$(IV) + NaOH \longrightarrow C_{10}H_{21}NHCH_2CHOHCH_2NH_2$$

$$base \xrightarrow{HCl} (chlorhydrate)$$

Si ce procédé est particulièrement adapté pour la préparation des éthers amino alcools lorsqu'on l'applique aux diamino-alcools, les rendements du procédé sont assez faibles.

Le rendement global de l'opération de synthèse desdits amino alcools par le procédé analogue à celui décrit dans le brevet américain 4 196 217 est de l'ordre de 35 %, en outre la purification des produits obtenus est particulièrement délicate et se prête mal à l'obtention d'un produit destiné à être utilisé notamment dans l'industrie pharmaceutique.

C'est pourquoi la présente invention propose un nouveau procédé permettant de préparer les diamino-alcools avec des rendements améliorés et dans des conditions permettant l'obtention de diamino-alcools très purs.

La présente invention concerne un procédé de préparation de diamino-alcools de formule :

$$R—NH—CH_2—CH(OH)—CH_2NH_2$$

dans laquelle R est un radical alkyle en $C_1$ à $C_{30}$, dans lequel :

a) on fait réagir une épihalohydrine avec une amine de formule $R—NH_2$ dans l'alcool isopropylique

ou l'éther de pétrole, à une température inférieure à 30 °C avec un excès de l'amine, pour obtenir un composé de formule :

$$R—NH—CH_2—CH(OH)—CH_2—X$$

dans laquelle X est un halogène,

b) ce produit étant mis à réagir avec l'ammoniac à une température comprise entre 100 et 200 °C, dans un alcool et sous pression.

Dans l'étape a) l'épihalohydrine est, par exemple, l'épichlorhydrine, dans ce cas X est le chlore. Lorsque le solvant est l'éther de pétrole, il s'agit d'éther de pétrole à 40-65 °C.

Dans l'étape b) l'alcool utilisé est de préférence le méthanol et la réaction peut être mise en œuvre dans un autoclave à une pression de 10 à 50 bars.

L'étape a) est conduite de préférence à température ambiante, et avec un excès de l'amine, ainsi avec 1 mole d'amine le rendement est de l'ordre de 50 % et avec un excès d'amine de 50 % en mole le rendement passe à 80 % en mole.

Le produit final I du procédé selon l'invention est isolé par tout procédé connu notamment par extraction liquide liquide, par exemple à l'aide d'un éther à partir d'une phase aqueuse basique, l'amine obtenue étant cristallisée de la phase éthérée sous forme de bichlorhydrate.

Afin d'améliorer encore le rendement d'amination du composé de formule II, on utilise l'ammoniac en présence d'un catalyseur notamment un halogénure d'ammonium, en particulier le bromure d'ammonium $NH_4Br$.

L'un des intérêts du procédé selon la présente invention est que le dérivé chloré II est un solide cristallisable dans l'isopropanol, ceci permet la séparation aisée du composé II et en outre, ce phénomène déplace la réaction chimique dans le sens souhaité.

Les composés de départ mis en œuvre dans ce procédé sont connus ou peuvent être préparés par des procédés connus.

Des essais comparatifs de préparation des composés de formule I par le procédé de la technique antérieure et par le procédé de l'invention montrent que le rendement qui est de l'ordre de 35 % pour la technique antérieure peut atteindre plus de 55 % dans le procédé de l'invention.

Les composés de formule I tels quels ou sous forme de sels non toxiques présentent des propriétés biocides notamment bactéricides qui les rendent aptes à être utilisés à titre d'agents antiseptiques dans des compositions pharmaceutiques ou bien comme conservateurs dans des compositions tant pharmaceutiques que cosmétiques ou autres. Ces composés peuvent également être utilisés seuls ou en combinaison pour stériliser des appareillages.

Les exemples ci-après sont destinés à mettre en évidence d'autres avantages et caractéristiques du procédé selon la présente invention.

## Exemple 1

Préparation du bichlorydrate de dodécyl-amino-3-hydroxy-2-amino-1-propane.

Dans un ballon de 1 litre, on introduit 185 g de dodécylamine dissout dans 500 ml d'alcool isopropylique. On additionne goutte à goutte 93 g d'épichlorhydrine fraîchement distillée en maintenant la température en dessous de 30 °C.

On maintient l'agitation pendant 4 heures à température ambiante.

Le dérivé chloré ainsi obtenu précipite : on le filtre et on le lave à l'isopropanol.

Rendement : 50 % F° : 62 °C.

Dans une bombe de Paar, on introduit 30 g d'une solution de méthanol saturé en ammoniac, puis par petites portions 10 g de dérivé chloré.

On ferme la bombe et on agite 4 heures à 90 °C. La solution obtenue est traitée par la soude à 10 % jusqu'à pH basique. Puis on extrait le dodécylamino-3-hydroxy-2-amino-1-propane à l'éther.

La phase éthérée est lavée, séchée sur sulfate de sodium anhydre et filtrée. On fait passer un courant d'acide chlorhydrique dans la solution éthérée. On met au réfrigérateur puis on essore les cristaux de bichlorhydrate de dodécylamino-3 hydroxy-2 amino-1 propane.

Rendement : 45 %

Point de fusion > 270 °C

Soluble dans l'eau.

## Exemples 2 à 4

En remplaçant dans le procédé décrit à l'exemple 1 la dodécylamine respectivement par la décylamine, la tétradécylamine et l'octadécylamine, on obtient les diamino-alcools correspondants dont les caractéristiques sont rappelées dans le tableau I ci-après.

## Exemples 5 à 8

Les composés des exemples 1 à 4 sont préparés sous forme de bichlorhydrate mais il est possible de remplacer l'acide chlorydrique par d'autres acides pharmaceutiquement acceptables notamment l'acide malonique, l'acide maléique, l'acide succinique ou l'acide méthane sulfonique. On obtient dans ces conditions les sels correspondants dont les caractéristiques sont rappelées dans le tableau I.

Tableau I

Composés R—NH—$CH_2$—CH(OH)$CH_2NH_2$,A

| Composé de l'ex. | R | A | F° | ANALYSE ELEMENTAIRE | |
|---|---|---|---|---|---|
| | | | | Calculé | Trouvé |
| 1 | $C_{10}H_{21}$ | 2 HCl | >270°C | C 51,49<br>H 10,56<br>N 9,24<br>Cl 23,43 | 52,72<br>11,01<br>9,27<br>23,57 |
| 2 | $C_{12}H_{24}$ | 2 HCl | >270°C | N 8,46<br>Cl 21,45 | 8,58<br>21,77 |
| 3 | $C_{14}H_{29}$ | 2 HCl | Sublimé ~250°C | N 7,80<br>Cl 19,78 | 7,85<br>19,95 |
| 4 | $C_{18}H_{37}$ | 2 HCl | 242°C | N 6,75<br>Cl 17,11 | 6,67<br>17,39 |
| 5 | $C_{12}H_{24}$ | 2 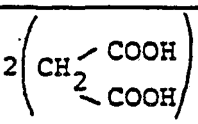 | ~140°C (mou) | C 54,08<br>H 9,01<br>N 6,02 | 54,23<br>9,49<br>6,03 |
| 6 | $C_{12}H_{24}$ | 2 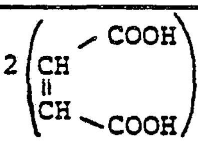 | 178°C | C 56,33<br>H 8,57<br>N 5,72 | 56,52<br>8,86<br>5,77 |
| 7 | $C_{12}H_{24}$ | 2 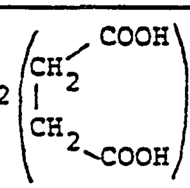 | 110°C | C 55,87<br>H 9,31<br>N 5,67 | 56,68<br>9,47<br>5,68 |
| 8 | $C_{12}H_{24}$ | 2($CH_3$ $SO_3H$) | ~100°C (mou) | C 45,33<br>H 9,33<br>N 6,22 | 44,70<br>9,33<br>6,40 |

Exemple 9

Dans une bombe de Paar, on introduit 10 g de bromure d'ammonium $NH_4Br$, 50 ml de méthanol saturé en ammoniac et par petites portions 14 g du dérivé chloré de l'exemple 1. On ferme la bombe et on agite 3 heures à 140 °C.

Après refroidissement, on évapore le méthanol et on traite la solution par la soude à 10 % jusqu'à pH basique et on extrait la diamine-alcool à l'éther comme cela a été décrit à l'exemple 1.

On obtient ainsi le composé du titre de l'exemple 1 avec un rendement de 70 %.

Exemple 10

L'activité bactéricide des composés selon la présente invention a été testée notamment sur P. Aeruginosa et Candida albicans.

Les résultats mesurés sont rappelés dans le tableau II ci-après en comparaison avec le chlorure de benzalkonium.

Tableau II

Activité des composés en ppm (par méthode interne)

| COMPOSE EXEMPLE | PSEUDOMONAS AERUGINOSA | | CANDIDA ALBICANS | |
|---|---|---|---|---|
| | Bactériostase | Bactéricidie | Bactériostase | Bactéricidie |
| 1 | compris entre 100 et 250 | - | 250 | - |
| 2 | 25 | 20 | 100 | - |
| 3 | 100 | - | 50 | - |
| 4 | > 500 | - | compris entre 10 et 50 | - |
| 5 | 25 | 100 | 75 | 500 |
| 6 | 25 | 250 | 75 | 750 |
| 7 | 25 | 500 | 75 | 1000 |
| 8 | 25 | 500 | 75 | 500 |
| Chlorure de Benzal-konium | 500 | - | 10 | - |

Exemple 11

L'activité bactéricide du composé de l'exemple 2 a été déterminé selon la norme AFNOR NF 72-151.
Produit : PVA 113

Mode opératoire déterminé à la suite de l'essai préliminaire

1. Nature des membranes utilisées et référence : Nitrate de cellulose 0,45 μ.
2. Diluant des suspensions bactériennes
Nature : Solution tryptone sel
Mode de préparation :
Nombre de lavages avec le diluant : 3
Volume du diluant utilisé pour chaque lavage : 50 ml.
3. Neutralisant(s) ajouté(s) au milieu de dénombrement et concentration : Néant
4. Résultats des essais préliminaires dans les conditions décrites ci-dessus :

| Concentrations essayées du produit | SOUCHES | Témoin | Produit |
|---|---|---|---|
| 1°/oo | Pseudomonas aeruginosa IPP A 22 | 111 | 120 |
| 1°/oo | Escherichia coli IPP54127 | 80 | 90 |
| 1°/oo | Staphylococcus aureus ATCC9144 | 126 | 120 |
| 1°/oo | Streptococcus faecalis ITCC10541 | 82 | 75 |
| 1°/oo | Mycobacterium smegmatis IPP7326 | 127 | 140 |

5. Validité de l'essai :

| | |
|---|---|
| Pseudomonas aeruginosa | 1 ‰ |
| E. coli | 1 ‰ |
| Staphylococcus aureus | 1 ‰ |
| Streptococcus faecalis | 1 ‰ |
| Mycobacterium smegmatis | 1 ‰ |

(Voir Tableau p. 7)

Résultats de la détermination de l'activité bactéricide

| SOUCHES | N Valeur comprise Entre 50 et 150 | X Concentration en pourcentage (m/v) au contact avec les bactéries | | | | | | pH |
|---|---|---|---|---|---|---|---|---|
| | | 0,02 ‰ | 0,05 ‰ | 0,1 ‰ | 0,2 ‰ | 0,5 ‰ | 1 ‰ | |
| Ps. aeruginosa IPP A 22 | 85 | + | 33 | 0 | 0 | 0 | 0 | 6,1 |
| E. coli IPP 54127 | 128 | + | + | 131 | 1 | 0 | 0 | 6,1 |
| St. aureus ATCC 9144 | 102 | + | + | + | 36 | 10 | 0 | 6,0 |
| Str. faecalis ATCC 10541 | 119 | + | + | 86 | 0 | 0 | 0 | 6,1 |
| M. smegmatis IPP 7326 | 150 | + | + | + | + | + | 118 | 6,0 |

+ = plus de 150 colonies

Concentration bactéricide du produit selon NF 72-151 : 1 ‰

**Revendications**

1. Procédé pour la préparation de composés de formule :

$$R—NH—CH_2—CH(OH)—CH_2NH_2$$

dans laquelle R est un radical alkyle en $C_1$ à $C_{30}$, dans lequel :

a) on fait réagir une épihalohydrine avec une amine de formule $R—NH_2$ dans l'alcool isopropylique ou l'éther de pétrole, à une température inférieure à 30 °C avec un excès de l'amine, pour obtenir un composé de formule :

$$R—NH—CH_2—CH(OH)—CH_2—X$$

dans laquelle X est un halogène,

b) ce produit étant mis à réagir avec l'ammoniac à une température comprise entre 100 et 200 °C, dans un alcool et sous pression.

2. Procédé selon la revendication 1, caractérisé en ce que dans l'étape de réaction avec l'ammoniac, l'alcool est le méthanol.

3. Procédé selon l'une des revendications 1 et 2, caractérisé en ce que la réaction avec l'ammoniac est conduite en présence d'un catalyseur halogénure d'ammonium quaternaire.

**Claims**

1. A process for the preparation of compounds corresponding to the formula :

$$R—NH—CH_2—CH(OH)—CH_2NH_2$$

wherein R is a $C_1$ to $C_{30}$ alkyl radical, in which

a) an epihalohydrin is reacted with an amine corresponding to the formula $R—NH_2$ in isopropyl alcohol or petroleum ether at a temperature below 30 °C with an excess of the said amine to obtain a compound corresponding to the formula :

$$R—NH—CH_2—CH(OH)—CH_2—X$$

in which X is a halogen,

b) this product is reacted with ammonia at a temperature between 100 and 200 °C, in an alcohol and under pressure.

2. A process according to claim 1, characterised in that the alcohol in the stage involving reaction with ammonia is methanol.

3. A process according to one of claims 1 and 2, characterised in that the reaction with ammonia is carried out in the presence of a quaternary ammonium halide catalyst.

**Patentansprüche**

1. Verfahren für die Zubereitung von Zusammensetzungen der Formel :

$$R—NH—CH_2—CH(OH)—CH_2NH_2$$

in der R ein $C_1$-bis $C_{30}$ Alkylrest ist, wobei man

a) ein Epihalofenhydrin mit einem Amin der Formel $R—NH_2$ in Isopropylalkohol oder Petroläther bei einer Temperatur unterhalb von 30 °C mit einem Amin-Überschuß umsetzt, um eine Verbindung der Formel

$$R—NH—CH_2—CH(OH)—CH_2—X$$

zu erhalten, in welcher X ein Halogenatom ist,

b) dieses Produkt mit Ammoniak bei einer Temperatur im Bereich zwischen 100 und 200 °C in Alkohol und unter Druck umsetzt.

2. Verfahren nach Anspruch 1, dadurch gekennzeichnet, daß der in der Stufe der Umsetzung mit Ammoniak verwendete Alkohol Methanol ist.

3. Verfahren nach Anspruch 1 oder 2, dadurch gekennzeichnet, daß die Umsetzung mit Ammoniak in Gegenwart eines quarternären Ammoniumhalogenids als Katalysator durchgeführt wird.